Europäisches Patentamt

European Patent Office

Office européen des brevets

(19)

(11) Publication number: **0 203 587**
**A2**

(12)

# EUROPEAN PATENT APPLICATION

(21) Application number: **86107244.5**

(22) Date of filing: **28.05.86**

(51) Int. Cl.⁴: **C 07 K 7/08,** G 01 N 33/531, G 01 N 33/574

(30) Priority: **30.05.85 US 739416**

(43) Date of publication of application: **03.12.86**
**Bulletin 86/49**

(84) Designated Contracting States: **AT BE CH DE FR GB IT LI NL SE**

(71) Applicant: **F. HOFFMANN-LA ROCHE & CO. Aktiengesellschaft, CH-4002 Basel (CH)**

(72) Inventor: **Chizzonite, Richard A., 10 Barnsdale Road, Clifton N.J. 07013 (US)**
Inventor: **Felix, Arthur M., 257 Central Avenue, West Caldwell N.J. 07006 (US)**
Inventor: **Furth, Mark E., 403 East 63rd Street, New York N.Y. 10021 (US)**

(74) Representative: **Lederer, Franz, Dr. et al, Vanderwerth, Lederer & Riederer Patentanwälte Lucile-Grahnstrasse 22, D-8000 München 80 (DE)**

(54) **Ras oncogene peptides and antibodies.**

(57) Novel ras oncogene polypeptides derived from the C-terminal variable region of different members of the p21ras protein family as well as monoclonal and polyclonal antibodies specific to such region are described. Such antibodies are useful for immunometric assays for the determination of the presence of each individual member of the p21ras oncogen protein family or combination thereof.

## Ras oncogene peptides and antibodies

Many mammalian tumor cells contain a mutated oncogene capable of inducing neoplastic transformation of rodent fibroblasts, when introduced by DNA-mediated gene transfer. The activated oncogenes most frequently detected by this assay are three members of a family, $ras^H$, $ras^K$, and $ras^N$ (Shimizu, K., et al., Proc. Natl. Acad. Sci. USA 80, 2112-2116 [1983]). Each of these genes encodes a protein of 188 or 189 amino acids, corresponding to about 21,000 daltons, called p21ras. The proteins bind guanine nucleotides and the $ras^H$ protein possesses GTPase activity (Gibbs, J.B., et al., Proc. Natl. Acad. Sci. USA 81, 5704--5708 [1984]). The amino acid sequences specified by the three ras genes are remarkably similar. The wild type proteins are identical over the first 86 amino acid residues, and pairs of ras proteins are about 85% homologous over the next 75 residues. However, the proteins differ dramatically in a segment of 15 amino acids (residues 171-185) located near the carboxyl terminus (for a review see Van Beveren, C. and Verma, I.M., Current Topics in Microbiology and Immunology, Vol. 123, p. 73-98 [1986]). The C-terminal variable region of each ras family member is highly conserved between rodents and humans. It has been proposed that the variable domain confers specificity for some physiological function of the p21ras proteins. A potential functional role for the variable domain is challenged by evidence suggesting that the carboxyl terminus of p21ras is proteolytically cleaved during post-translational processing (Shimizu, K. et al., Nature 304, 497-500 [1983]). However, more recently it has been found that the C-terminus of the $ras^H$ protein remains covalently intact during processing. Additional data suggest that the same is

Wä/5.5.86

true for the ras$^K$ protein as well.

Other groups have noted the presence of a putative endopeptidase target site just upstream of the variable domain in each ras protein. They proposed that the protein is cleaved at this site, but that a disulfide bridge involving a conserved cysteine residue at position 186 holds the C-terminal fragment to the rest of the molecule (Willumsen, B.M. et al., EMBO J. 3, 2581-2585 [1984]). This model predicts that reduction of mature p21ras should liberate a peptide of about 2,000 daltons. Again, more recent data indicates the failure to detect the release of such a peptide from ras$^H$ proteins. Thus, there is now strong positive evidence for the covalent integrity of the C-terminal domain of p21ras proteins. Since it is apparent that the variable region does remain intact in the ras protein family, it should be possible to make use of the sequence difference among the various members of the p21ras family of proteins, i.e., ras$^H$, ras$^N$, ras$^{4KA}$ and ras$^{4KB}$, to provide selective reagents which can differentiate between these proteins and which can then be used to monitor whether or not the oncogenes are on or off in a subject. In this manner, it will be possible to determine whether individual subjects are in high risk groups for neoplastic diseases and also to monitor stages of neoplastic diseases in patients already having same. Additionaly it would be possible to assess the stage of development of cancers and to monitor patients during therapy (Niman, H.L., Hybridoma, Vol. 4, No. 1, 1985, p. 75).

The present invention relates to the preparation of novel polypeptides having an amino acid sequence derived from the variable regions of the different members of the p21ras protein family, the utilization of such polypeptides to prepare immunogenic compositions utilizing such polypeptides covalently linked to immunogenic carrier materials, the production of antibodies elicited by such polypeptides which antibodies are specific to the ras oncogene from which the polypeptide sequence was derived and to immunoassay methods employing such antibodies to determine the presence of each individual p21ras oncogene family member.

A first aspect of the present invention relates to the preparation of a series of polypeptides derived from the variable region in the carboxy terminal portion of human p21ras protein. The polypeptides of the present invention comprise the following specific sequences:

$H_2$N-Leu-Asn-Pro-Pro-Asp-Glu-Ser-Gly-Pro-Gly-Cys-      (I)
Met-Ser-Cys-Lys-Cys-Val-Leu-Ser-OH

$H_2$N-Lys-Leu-Asn-Pro-Pro-Asp-Glu-Ser-Gly-Pro-Gly-Cys      (II)
Met-Ser-Cys-Lys-Cys-Val-Leu-Ser-OH

$H_2$N-Lys-Leu-Asn-Ser-Ser-Asp-Asp-Gly-Thr-Gln-Gly-Cys-    (III)
Met-Gly-Leu-Pro-Cys-OH

$H_2$N-Ile-Ser-Lys-Glu-Glu-Lys-Thr-Pro-Gly-Cys-Val-Lys-    (IV)
Ile-Lys-Lys-Cys-OH

$H_2$N-Met-Ser-Lys-Asp-Gly-Lys-Lys-Lys-Lys-Lys-Lys-      (V)
Ser-Lys-Thr-Lys-Cys-OH

and derivatives thereof.

Sequences I and II represent respectively p21ras[H] (171-189) and p21ras[H] (170-189) polypeptides. Sequence III represents p21ras[N] (170-186) polypeptide. Sequence IV represents p21ras[K4A] (171-186) polypeptide. Finally, sequence V represents p21ras[K4B] (170-185) polypeptide.

The above specified sequences can be synthesized utilizing conventional peptide synthesis procedures well known in the art. Such procedures include solution phase synthesis and solid phase synthesis applying the methodology developed by R.B. Merrifield as described in J. Am. Chem. Soc., 85, 2149-2154 (1963). The preferred methodology for synthesizing the peptides of the present invention is the solid phase method which employs cyclic additions of amino acids to the carboxy terminal amino acid of the peptide desired to be synthesized, covalently bound to a conventional solid phase synthesis resin. Suitable resins for this purpose are articles of commerce and include for example cross-linked polystyrene resins. Such resins can now be obtained commercially with the desired carboxy terminus amino acid already covalently linked to the resin. The added amino acids can be protected with conventionally employed side-chain protecting groups. Suitable side-chain protecting groups for this purpose include benzyl, benzyloxycarbonyl, chlorobenzyl, p-chloro-benzyloxycarbonyl and p-toluenesulfonyl or dimethyl benzyl.

In the last step of such a solid phase synthesis the peptide is deprotected and released from the resin by procedures well known in the art e.g. by methods as described in detail in Example 1.

Similarly, if liquid phase synthesis is used, the polypeptide is obtained by removal of the protecting groups by conventional means e.g. by methods as described in Houben-Weyl, "Methoden der organischen Chemie", Volume XV "Synthese von Peptiden".

Thus, in this manner, polypeptides I-V defined above were prepared and constitute preferred embodiments of the present invention.

The above described polypeptides can be employed as immunogens to prepare antibodies.

Further aspects of the present invention therefore relate to immunogenic compositions and their preparation, to antibodies elicited by these immunogenic compositions and to immunometric assays for the human p21ras polypeptides mentioned above.

Such immunogenic compositions are readily obtained by covalently bonding each of the above described polypeptides to a conventional immunogenic carrier material. The term "immunogenic carrier material" is meant to include those materials which have the property of independently eliciting an immunogenic response in a host animal and which can be covalently coupled to the aforesaid polypeptides either directly by the formation of peptide or ester bonds between free carboxyl, amino or hydroxyl groups in the aforesaid polypeptides and corresponding groups on the immunogenic carrier material or alternatively by bonding through a conventional bifunctional linking group.

The covalent coupling of the polypeptides of the invention to the immunogenic carrier material can be carried out in a manner well known in the art. Thus, for example, for direct covalent coupling, it is possible to utilize a carbodiimide, most preferably dicyclohexyl carbodiimide or 1-ethyl-3-(3-dimethylaminopropyl) carbodiimide as coupling agent. In such direct coupling, it is desirable to utilize a slightly acidic reaction medium for this step, e.g., a medium having a pH in the range of from about 3 to 6.5, most preferably in the range of from about 4 to 6.5.

A suitable bifunctional linking group for effecting coupling is a $C_{2-7}$ dialkanal such as glutaraldehyde. Such coupling in this alternative embodiment can conveniently be carried out using the conditions described by S. Avrameas in Immunochemistry $\underline{6}$, 43-52 (1969).

Still another preferred reagent for use in coupling of the polypeptides of the present invention to the immunogenic carrier material is m-maleimidobenzoyl N-hydroxysuccinimide (MBS) which can be utilized at room temperature in an aqueous soluble solvent such as for example N,N-dimethylformamide (DMF). It is preferred to utilize the immunogenic carrier material dissolved in a suitable phosphate buffer at pH 7.2.

The resulting immunogenic compositions can be utilized without further purification or, if desired, after purification by procedures well known in the art such as for example dialysis to remove any unreacted peptide and coupling reagents or alternatively by use of column chromatography on a suitable column (e.g. Sephadex$^{TM}$ G-25 gel chromatography).

Suitable carrier materials which can be used in the preparation of the immunogenic compositions of the instant invention include proteins, natural or synthetic polymeric compounds such as polypeptides, e.g., polylysine or copolymers of amino acids; polysaccharides; and the like. Particularly, preferred carrier materials are proteins and polypeptides, especially proteins.

The identity of the protein utilized in the preparation of an immunogenic composition of the instant invention is not narrowly critical. Examples of suitable proteins include mammalian serum proteins such as, for example, human gammaglobulin, human serum albumin, bovine serum albumin, methylated bovine serum albumin, rabbit serum albumin, bovine gammaglobulin, bovine thyroglobulin and equine gammaglobulin or nonmammalian proteins such as hemocyanin, most preferably keyhole limpet hemocyanin. Other suitable proteins will be suggested to one skilled in the art.

The immunogenic compositions of the present invention may be utilized to induce formation of antibodies specific to the respective H-ras, N-ras and K-ras p21 proteins in host animals by injecting the immunogenic composition in such a host, preferably using an adjuvant. Improved titers can be obtained by repeated injections over a period of time. Suitable host animals for this purpose include mammals such as rabbits, guinea pigs, horses, goats, rats, mice, cows, sheep, etc. The resulting antisera contain antibodies which will selectively complex with the respective oncogene proteins. Such sera can be utilized per se in carrying out assays for such oncogene protein, or if desired, the antibody can be concentrated by utilizing procedures well known in the art such as, for example, by ammonium sulfate precipitation followed by gel chromatography. In an alternative embodiment of the present invention, monoclonal antibodies useful in the detection of the indicated p21ras oncogene proteins may be obtained by known methodologies available in the art such as, for example, those described by C. Milstein and G. Köhler in Nature, 256, 495-497, (1975). In such procedure, the immunogenic compositions of the present invention are injected in a mouse or a rat. The host animal is subsequently sacrificed and cells taken from its spleen are fused with myeloma cells. The result is a hybrid cell, referred to as a "hybridoma", that reproduces in vitro. The population of hybridomas are screened to isolate individual clones, each of which secretes a single antibody species to the antigen polypeptide which was used in the immunogenic composition. The individual antibody species obtained in this way are each the product of a single B cell from the immunized animal, generated in response to a specific antigenic site recognized on the immunogenic substance. In this instance, since a subsequence of the p21 protein was employed, the antibody will also be specific to the full p21 protein containing this subsequence.

The different hybridoma cell lines are then screened to identify those that produce antibody to the desired antigen. The antibodies produced by the different hybridoma cell lines are preferably screened to identify those having the best affinity and avidity for the immunogenic substance used for the production of the hybridoma cell lines.

The polyclonal or monoclonal antibodies produced in accordance with the present invention can be utilized in any conventional immunometric assay for use in detecting the presence of the respective p21ras proteins in test samples. In one such procedure, known amounts of a sample to be assayed, the specific antibody of the present invention and labelled p21ras polypeptide or protein are mixed together and allowed to stand. The antibody-antigen complex is separated from the unbound reagents by procedures known in the art, i.e., by treatment with ammonium sulfate, polyethylene gycol, second antibody either in excess or bound to an insoluble support. Suitable insoluble supports include polymers such as Kynar$^{TM}$, dextran coated charcoal and the like. The concentration of the labelled ras polypeptide or protein is determined in either the bound or unbound phase and the specific p21ras protein content of the sample can then be determined by comparing the level of labelled component observed to a standard curve in a manner known per se. A suitable standard curve can be obtained by mixing known amounts of p21ras protein with fixed amounts of labelled p21ras protein and the p21ras specific antibody of the present invention and determining the degree of binding for each such known amount. Since the antibodies of the present invention can distinguish between the respective H, N, K4A and K4B forms of the p21ras protein, it is possible to determine the presence of each of these individual forms of the ras oncogene protein even in the presence of mixtures.

Two different monoclonal antibodies to a specific p2lras oncogene protein, which antibodies do not interfere with the binding of each other to the antigen, may be employed in a two-site immunometric assay procedure. Suitable homogenous and heterogenous two-site immunometric assay procedures are described in U.S. Patent 4,376,110.

Yet another immunometric methodology which can be employed in the practice of the present invention involves the use of an immunoblot technique (so-called Western blot analysis). In this technique, samples containing the p2lras protein are mixed with SDS sample buffer (Laemmli, U.,K., Nature 227 680-685 [1970]) with or without 2-mercaptoethanol as sulfhydryl reducing reagent, boiled and electrophoresed on polyacrylamide gels. Proteins are electrophoretically transferred to nitrocellulose filters using a "Transblot" apparatus following the procedure described by H. Towbin et al., Proc. Natl. Acad. Sci. USA 76, 4350-4354 (1979). After preincubation with buffered bovine serum albumin, the filters are incubated overnight with antisera to the desired human p2lras polypeptide. The filters are then washed and incubated sequentially with appropriate labelled antisera directed against the IgG of the species in which the ras specific antibody was developed. Such second antibody can be labelled with any conventional label such as a radioisotope or preferably with an enzyme such as with peroxidase. The filters may then be washed again and either autoradiographed or incubated with an appropriate substrate for the enzyme such as, for example, with diaminobenzidine until the protein bands are observed. The absence of color bands with any specific antibody to human p2lras protein would indicate the absence of such protein in the test sample. If one desires only knowing whether or not ras oncogenes product protein were present in the test sample, then combinations of the specific antibodies may be employed. If, on the other hand, the presence or absence of a specific p2lras protein is to

be determined, then a panel of the individual antibodies specific for the individual ras proteins would be employed on repetitive test samples.

The antibodies of the present invention may also be utilized in heterogenous "sandwich" type assays. In such a heterogenous assay, an unlabelled antibody, preferably a monoclonal antibody, is used to extract the test protein substance from the sample , such antibody is immobilized on any of the conventional supports used in immunometric assays. Among these supports, there is included, filter paper, plastic beads or test tubes made from polyethylene, polystyrene, polypropylene or other suitable material. Also useful for this purpose are particulate materials such as agarose, cross-linked dextran and other polysaccharides. The techniques for such bonding are well known to those skilled in the art. For example, antibodies may be bound to polysaccharide polymers using the process described in U.S. Patent No. 3,645,852.

Labeled specific antibody used in the present invention may be provided with the same labels used in immunometric assays known in the art. Among these may be mentioned fluorogenic labels for detection by fluorimetry as described in U.S. Patent No. 3,940,475 and enzymatic markers as described in U.S. Patent No. 3,654,090. It is also possible to utilize a radiolabelled antibody such as, for example, $^{125}$I using, for example, the procedure of W.M. Hunter and F.C. Greenwood in Nature (London) 194, 495-496 (1962) or that of G.S. David and R.A. Reisfeld in Biochemistry, 13, 1014-1021, (1974).

In a typical heterogenous sandwich assay, the amount of labelled antibody associated with the insoluble sandwich complex is determined by examination of the insoluble carrier material by suitable means. However, it is also possible to relate the presence or absence of the test protein in the fluid sample being assayed to the amount of labelled antibody which does not react during the assay and remains in a soluble form.

In yet another embodiment of the present invention, the antibodies of the invention can be used for antibody affinity chromatography purification of the respective p21ras proteins for which they are specific binding partners. For this purpose, the antibodies are immobilized on a matrix in a manner known per se, suitably covalently bound to a suitable antibody affinity chromatography matrix such as a cross-linked agarose (e.g. Sepharose$^{TM}$ 4B, commercially available from Pharmacia). The antibody affinity chromatography purification of human p21ras protein containing solutions, may be performed according to any of the well known methods, either batchwise or, preferably, using the matrix-immobilized antibody arranged on a column.

## EXAMPLE 1

## Synthesis of H-ras T-24 oncogene-encoded nonadecapeptide p21ras$^H$ (171-189).

Solid-phase peptide synthesis was carried out in an upright vessel clamped to a Model S-500 shaker equipped with an RD-20 shaker head (Kraft Apparatus, Inc., Minneola, N.Y.) using a manual procedure. Ser(Bzl)-1% cross-linked poly-styrene (200-400 mesh) resin [3.0 g, 0.21 mmol/g-resin, 0.63 mmol (purchased from BACHEM, Torrance, CA)] was subjected to 18 cycles of solid peptide synthesis following the sequence of p21ras$^H$ from position 186 to 171 using the preformed symmetric anhydride method (Merrifield, R.B., supra). The α-amino groups were protected by the t-butyloxycarbonyl group, whereas the side-chain groups were protected as follows: Asp, Glu, Ser (benzyl); Cys (3,4-dimethylbenzyl); Lys (2-chlorobenzyloxycarbonyl). 4.4 g (0.63 mmol) of the protected nonadecapeptide [p21ras$^H$ (171-189)]-resin were

obtained. 1.0 g (0.143 mmol) of this protected peptide-resin was treated with anhydrous liquid hydrogen fluoride (HF) (10ml containing 10% 1,2-ethanedithiol) at 0° for 1h and evaporated (high-vac, CaO trap). The crude peptide and resin mixture was triturated with ethyl acetate (EtOAc), extracted with trifluoroacetic acid (TFA), evaporated and the residue triturated with ether and dried. The crude material (0.515 g) was dissolved in 10 ml of distilled $H_2O$, filtered and loaded onto a Whatman Partisil™ M-20 ODS-3 column (2 x 50 cm).

The column was eluted (6.0 ml/min) with a solvent system consisting of (A) $H_2O$ (containing 0.1% TFA) and (B) acetonitrile (containing 0.1% TFA) in a linear gradient mode of 10-35% (B) in 180 min. Fractions were collected (2 min/fraction) and aliquots analyzed using an analytical hplc system. The product which emerged in fractions 61-65 were combined, evaporated and lyophilized to give the pure nonadecapeptide p21ras$^H$ (171-189). Yield: 42 mg (15.2%). The product was shown to be homogeneous by analytical hplc and gave the expected amino acid composition (hydrolysis in 6N aqueous HCl, 110°, 24h): Asp, 1.98; Ser, 2.71; Glu, 1.03; Pro, 3.15; Gly, 2.08; Val, 1.02; Leu, 1.93; Lys, 1.10; Met, 0.95; Cys, 2.74.

EXAMPLE 2

Synthesis of H-ras T-24 oncogene-encoded eicasapeptide p21ras$^H$ (170-189).

2,17 g (0.310 mmol) of the protected nonadecapeptide [p21ras$^H$ (171-189)]-resin obtained in Example 1 was subjected to one cycle of peptide synthesis with $N^\alpha$-Boc-$N^\epsilon$-2-chloro-benzyloxycarbonyl-Lys. A 1 g-portion (0.143 mmol) of the product was cleaved with liquid HF (as in Example 1). Yield: 525.6 mg. The crude product was subjected to hplc purification (as in Example 1) and 24.4 mg of the eicosapeptide p21ras$^H$ (170-189) (yield: 8.3%) was obtained.

The product was homogeneous by analytical hplc and gave the expected amino acid composition (hydrolysis: 6N aqueous HCl, containing 1% thioglycolic acid (TGA); 150°; 1h): Asp, 1.90; Ser, 2.30; Glu, 1.09; Pro, 2.81; Gly, 2.00; Val, 0.94; Leu, 1.80; Met, 1.02; Lys, 1.97; Cys, 2.71.

### EXAMPLE 3

### Synthesis of N-ras T-24 oncogene-encoded heptadecapeptide p21ras$^N$ (170-186).

Cys(DMB)-1% cross-linked polystyrene resin (2.0 g; 0.48 mmol/g-resin; 0.96 mmol) was subjected to 16 cycles of solid phase peptide synthesis following the sequence of p21ras$^N$ from position 185 to 170. A portion of the resultant protected peptide resin (1 g; 0.26 mmol) was treated with anhydrous liquid HF (as in Example 1). Yield: 385 mg. The crude peptide was subjected to hplc purification (as in Example 1). Gradient time: 150 min; the desired product emerged in fractions 50-51. These fractions were combined evaporated and lyophilized. Yield: 20 mg (4.4%) of the heptadecapeptide p21ras$^N$ (170-186), which was shown to be essentially homogeneous by analytical hplc. Amino acid composition (hydrolysis in 6N aqueous HCl/1% TGA; 150°; 1h): Asp, 2.00; Thr, 1.08; Ser, 1.65; Glu, 2.06; Pro, 1.09; Gly, 3.00; Met, 1.02; Leu, 1.99; Lys, 1.00. Cys (Ellman test), 2.05.

### EXAMPLE 4

### Synthesis of K-ras T-24 oncogene[exon IVa]-encoded hexadecapeptide p21ras$^{K4A}$ (171-186).

Cys (DMB)-1% cross-linked polystyrene resin (2.0 g; 0.48 mmol/g-resin; 0.96 mmol) was subjected to 15 cycles of solid phase peptide synthesis following the sequence of p21ras$^{4KA}$ from position 185 to 171, treated with TFA and dried to give 6.52 g (wet weight) of the side-chain protected peptide resin. A portion (1 g; 0.147 mmol) was cleaved with HF (as in example 1) and subsequent workup gave 374.6 mg of crude hexadecapeptide p21ras$^{K4A}$ (171-186). The crude material was subjected to hplc purification [as in Example 1; Gradient: 5-30% (B)/180 min]. The product emerged in fractions 43-49 which were combined, evaporated and lyophilized. Yield: 101 mg (38.4%) of hexadecapeptide p21ras$^{K4A}$ (171-186), which was judged to be essentially homogeneous by analytical hplc. Amino acid composition (hydrolysis in 6N aqueous HCl/4% TGA; 150°; 1h): Thr, 0.99; Ser, 0.87; Glu, 2.07; Pro, 1.03; Gly, 1.07; Val, 0.97; Ile, 1.89; Lys, 5.10; Cys (as cysteic acid), 1.72. Cys (Ellman test, 1.96).

EXAMPLE 5

Synthesis of K-ras T-24 oncogene[exon IVb]-encoded hexadeca-peptide p21ras$^{K4B}$ (170-185)

Cys(DMB)-1% cross-linked polystyrene resin (2.0 g; 0.48 mmol/g-resin; 0.96 mmol) was subjected to 15 cycles of solid phase peptide synthesis following the sequence of p21ras$^{4KB}$ from position 184 to 170, treated with TFA and dried to give 4.71 g of the side-chain protected peptide resin. Anhydrous HF cleavage (as in example 2) of a 1 g (0.20 mmol) portion, and subsequent workup gave 300.5 mg of crude hexadecapeptide p21ras$^{K4B}$ (170-185). The crude material was subject to hplc purification [as in Example 1; Gradient: 5-20% (B)/150 min] and the fractions (20-30) containing the product were combined, evaporated and lyophilized. Yield: 64 mg (17.3%) of the hexadecapeptide p21ras$^{K4B}$ (170-185). The product was shown to be essentially homogeneous by analytical hplc. Amino acid composition (hydrolysis in 6N aqueous HCl/1% TGA; 150°; 1h): Asp, 0.99; Thr, 1.02; Ser, 1.83; Cys (as cysteic acid), 0.88; Gly, 0.99; Met, 1.05; Lys, 9.33. Cys (Ellman), 1.04.

## EXAMPLE 6

Conjugation of the nonadecapeptide p21ras[H] (171-189) via the KLH-MBS-procedure

Keyhole limpet hemocyanin (KLH) (16 mg in 50%, glycerol solution) was added to buffer solution (pH 7.2; 1.2 ml) made of 0.3 ml each of $Na_2HPO_4$ (8 mM); KCl (1.6 mM); NaCl (140 mM) and $KH_2PO_4$ (1.1 mM) and stirred magnetically at room temperature. A solution of m-maleimidobenzoyl-N- -hydroxysuccinimide (MBS) in DMF (0.6 ml) was slowly added (dropwise) and the slightly turbid reaction mixture stirred for 30 min at room temperature. The reaction product, KLH-MB, was then passed through a Sephadex[TM] G-25 column (1.2 x 50 cm) which had been previously equilibrated with 0.05M sodium phosphate buffer (pH 6.0). The column was eluted with the same buffer (flow rate: 1 ml/min) and fractions collected (1 min/fraction). The fractions containing the KLH-MB complex (fractions 20-44) were combined (20 ml; 10.3 OD units at a wavelength $\lambda$ = 280 nm). A portion of the KLH-MB product (5 mL, 2.06 OD) was reacted with 5 mg nonadecapeptide p21ras[H] (171-189) dissolved in 2 ml of 0.05M sodium phosphate buffer. The pH was adjusted to 7-7.5 with 1N NaOH and the reaction stirred for 3h at room temperature. The solution was dialyzed against phosphate buffered saline (pH 7.2) with frequent changes over a 40h period and then lyophilized. Yield of immunogenic composition 37 mg (containing phosphate buffered saline salts).

## EXAMPLE 7

Conjugation of the nonadecapeptide p21ras$^H$ (171-189) with thyroglobulin

Bovine thyroglobulin (6.69 mg, 10 nanomoles) was dissolved in 10 ml of 0.1M NaH$_2$PO$_4$, pH 7.2 and 9.6 mg (5 μmol) of nonadecapeptide p21ras$^H$ (171-189) was added. After all the peptide had dissolved, 0.2 mL of a solution of glutaraldehyde [Sigma Grade I, 25% (aqueous)] was added and the reaction continuously stirred at 25° for 2h. The mixture was extensively dialyzed against 0.001M NaH$_2$PO$_4$ (pH 7.2) buffer and lyophilized. Yield of immunogenic composition 17 mg (containing phosphate salts).

## EXAMPLE 8

Preparation of antibodies specific for the C-terminus of p21ras proteins or polypeptides.

Peptides corresponding to the carboxyl termini of p21ras$^H$, p21ras$^K$ and p21ras$^N$ synthesized as above by solid phase techniques were coupled to bovine thyroglobulin by the glutaraldehyde procedure as described in Example 7 or to keyhold limpet hemocyanin by the MBS procedure (Liu, F.-T. et al., Biochemistry 18, 690-697, [1979]) as described in Example 6. The conjugates were lypholized and resuspended at a concentration of 3 mg conjugate per ml of phosphate buffered saline (20 mM sodium phosphate, pH 7.4, 0.15 M NaCl) before use. The conjugants contained between 550 and 606 μg peptide per mg of conjugate.

Balb/C mice and Lewis rats were immunized according to the following schedule.

Immunization Protocol

Day 0     0.2 mg conjugate (1:1 mix with Freund's complete adjuvant) intraperitoneal injection (i.p.)

Week 8    2nd immunization (booster) 0.2 mg conjugate (1:1 mix with Freund's complete adjuvant) inject i.p.

Week 11   Bleeding

Week 27   3rd immunization (booster) 0.1 mg conjugate (1:1 mix with Freund's incomplete adjuvant) inject i.p.

Week 30   Bleeding

The animals were bled at the times indicated to determine circulating titers of anti-p21ras antibodies. The titers and specificity of the antisera were determined by direct binding ELISA and by an immuno-blot technique (so--called Western blot analysis). The results are shown in Tables 1 and 2.

## Table 1

Reactivities of mouse antisera specific for the C-terminus of p21ras proteins and polypeptides.

| Animal | Immunogen Peptide - Carrier | End-point titer (pooled sera from 3 mice) | Western Blots |
|--------|------------------------------|--------------------------------------------|----------------|
| Mouse | 1. p21ras$^H$ C terminus | | |
| | a.a. 171-189 - KLH (93-2a) | $10^{-6}$ | − |
| | a.a. 170-189 - KLH (93-2a') | $10^{-5}$ | |
| | a.a. 171-189 - TG (95-2a) | $10^{-6}$ | |
| | a.a. 170-189 - TG (95-2a') | $10^{-5}$ | |
| | 2. p21ras$^{K4A}$ C terminus | | |
| | a.a. 171-186 - KLH (93-2c) | − | |
| | a.a. 171-186 - TG (95-2C) | − | |
| | 3. p21ras$^{K4B}$ C terminus | | |
| | a.a. 171-185 - KLH (127-2d) | $10^{-4}$ | + |
| | a.a. 171-185 - TG (124-2d) | $10^{-3}$ | + |
| | 4. p21ras$^N$ C terminus | | |
| | a.a. 171-186 - KLH (93-2b) | $10^{-5}$ | |
| | a.a. 171-186 - TG (95-2b) | $10^{-5}$ | − |

Table 2

Reactivities of rat antisera specific for the C terminus of p21ras proteins and polypeptides.

| Animal | Immunogen Peptide – Carrier | End-point Titer (highest in pair) | Western Blots |
|--------|------------------------------|-----------------------------------|----------------|
| Rat | 1. p21ras$^H$ C terminus | | |
| | a.a. 171-189 – KLH (93-2a) | $10^{-6}$ | + |
| | a.a. 170-189 – KLH (93-2a') | $10^{-5}$ | + |
| | a.a. 171-189 – TG (95-2a) | $10^{-6}$ | + |
| | a.a. 170-189 – TG (95-2a') | $10^{-6}$ | + |
| | 2. p21ras$^{K4A}$ C terminus | | |
| | a.a. 171-186 – KLH (93-2c) | $10^{-4}$ | |
| | a.a. 171-186 – TG (95-2c) | $10^{-3}$ | |
| | 3. p21ras$^{K4B}$ C terminus | | |
| | a.a. 171-185 – KLH (127-2d) | $10^{-6}$ | + |
| | a.a. 171-185 – TG (124-2d) | $10^{-2}$ | |
| | 4. p21ras$^N$ C terminus | | |
| | a.a. 171-186 – KLH (93-2b) | $10^{-6}$ | |
| | a.a. 171-186 – TG (95-2b) | $10^{-6}$ | – |

EXAMPLE 9

Immunoblotting of ras proteins with anti-peptide sera and Y13-259 (a broad specificity ras monoclonal antibody used as a positive control) was carried out against lysates prepared from a series of cell lines, each of which expresses ras genes at a high level. Unlabelled, exponentially growing cells were lysed by incubation for 10 min at 4° C in TNE-buffer (20mM Tris-HCl, 100 mM NaCl, 1 mM EDTA, pH 7.4) containing 1% (v/v) Triton$^{TM}$ X-100, 0.5% (w/v) Na-deoxycholate, and 2 mM phenylmethylsulfonyl-fluoride. Samples were centrifuged at 100,000 x g for 30 min, and supernatants were collected and stored at -80° C. Relative protein concentrations were determined by a colorimetric assay using a BioRad assay kit, with bovine serum albumin (BSA) as standard. Portions of lysate containing 0.05 mg of total protein were mixed with equal volumes of 2x SDS sample buffer with or without 2-mercaptoethanol as sulfhydryl reducing reagent, boiled for 3 min, and electrophoresed on 12% polyacrylamide gels. Proteins were electrophoretically transferred to nitrocellulose filters (0.22 micron pore) using a "Transblot" apparatus essentially as described by H. Towbin et al., (supra). After preincubation for 3 h at 4° C with 3% BSA in TNE buffer, the filters were incubated overnight at 4° with monoclonal antibody Y13-259 at 10 μg/ml or with anti-peptide serum diluted 1:10 in TNE buffer with 3% BSA. The filters were washed, and incubated sequentially for 2 h with antiserum to rat IgG diluted 1:2000 and with peroxidase-conjugated, affinity-purified antibodies to rabbit IgG, prepared in goats (Boehringer-Mannheim), diluted 1:800. The filters were washed and incubated with diaminobenzidine substrate until brown protein bands were observed.

The broad specificity ras monoclonal antibody Y13-259 reacts with all the ras proteins tested. Rat antiserum to human p21ras$^{H}$ reacts with p21 encoded by the v-ras gene of HaMSV and by human p21ras$^H$, but not with p21 encoded by the v-ras$^K$ gene of KiMSV, human p21ras$^K$ or human p21ras$^N$.

Results of immunoblotting with a murine antiserum to ras$^{K4B}$ C-terminal peptide show that the serum reacts with denatured and reduced p21ras from the murine hemopoietic cell line 416B, which expresses very high levels of ras$^K$ protein. The ras$^K$ protein expressed in 416B cells appears to be a mixture of the 4A and 4B types. The murine antiserum to ras$^{K4B}$ C-terminal peptide reacts with human ras$^K$ protein but does not react detectably with the p21 ras encoded by KiMSV. The anti-serum failed to react with p21 proteins encoded by ras$^H$ and ras$^N$.

Claims:

1. A polypeptide selected from the group consisting of:

H₂N-Leu-Asn-Pro-Pro-Asp-Glu-Ser-Gly-Pro-Gly-Cys-    (I
   Met-Ser-Cys-Lys-Cys-Val-Leu-Ser-OH

H₂N-Lys-Leu-Asn-Pro-Pro-Asp-Glu-Ser-Gly-Pro-Gly-Cys    (II
   Met-Ser-Cys-Lys-Cys-Val-Leu-Ser-OH

H₂N-Lys-Leu-Asn-Ser-Ser-Asp-Asp-Gly-Thr-Gln-Gly-Cys-    (II]
   Met-Gly-Leu-Pro-Cys-OH

H₂N-Ile-Ser-Lys-Glu-Glu-Lys-Thr-Pro-Gly-Cys-Val-Lys-    (I\
   Ile-Lys-Lys-Cys-OH

H₂N-Met-Ser-Lys-Asp-Gly-Lys-Lys-Lys-Lys-Lys-    (\
   Ser-Lys-Thr-Lys-Cys-OH

and of derivatives thereof.

2. A polypeptide derivative comprising a compound of claim 1 in side-chain and N-terminal protected form with the carboxyl terminal being covalently linked to a polystyrene solid phase synthesis resin.

3. An immunogenic composition suitable for eliciting formation of antibodies specific for a p21ras polypeptide or protein in a host, said immunogenic composition comprising a polypeptide of claim 1 covalently bound to an immunogenic carrier material.

4. An antibody selective for human p21ras$^H$ polypeptide and non-crossreactive with human p21ras$^N$, p21ras$^{K4A}$ and p21ras$^{K4B}$ polypeptides.

5. The antibody of claim 4 elicited with an immunogenic composition according to claim 3 said immunogenic composition comprising a polypeptide having the sequence:

$H_2$N-Leu-Asn-Pro-Pro-Asp-Glu-Ser-Gly-Pro-
Gly-Cys-Met-Ser-Cys-Lys-Cys-Val-Leu-Ser-OH.

6. The antibody of claim 4 elicited with an immunogenic composition according to claim 3 said immunogenic composition comprising a polypeptide having the sequence:

$H_2$N-Lys-Leu-Asn-Pro-Pro-Asp-Glu-Ser-Gly-Pro-
Gly-Cys-Met-Ser-Cys-Lys-Cys-Val-Leu-Ser-OH.

7. An antibody selective for human p21ras$^N$ polypeptide and non-crossreactive with human p21ras$^H$, p21ras$^{K4A}$ and p21ras$^{K4B}$ polypeptides.

8. The antibody of claim 7 elicited with an immunogenic composition according to claim 3 said immunogenic composition comprising a polypeptide having the sequence:

$H_2$N-Lys-Leu-Asn-Ser-Ser-Asp-Asp-Gly-Thr-
Gln-Gly-Cys-Met-Ser-Cys-Lys-Cys-OH.

9. An antibody selective for human p21ras$^{K4A}$ polypeptide and non-crossreactive with human p21ras$^H$, p21ras$^N$ and p21ras$^{K4B}$ polypeptides.

10. The antibody of claim 9 elicited with an immunogenic composition according to claim 3 said immunogenic composition comprising a polypeptide having the sequence:

$H_2$N-Ile-Ser-Lys-Glu-Glu-Lys-Thr-Pro-Gly-
Cys-Val-Lys-Ile-Lys-Lys-Cys-OH.

11. An antibody selective for human p21ras$^{K4B}$ polypeptide and non-crossreactive with human p21ras$^{H}$, p21ras$^{N}$ and p21ras$^{K4A}$ polypeptides.

12. The antibody of claim 11 elicited with an immunogenic composition according to claim 3 wherein said immunogenic composition comprising a polypeptide having the sequence:

$$H_2N-Met-Ser-Lys-Asp-Gly-Lys-Lys-Lys-Lys-Lys-$$
$$Lys-Ser-Lys-Thr-Lys-Cys-OH.$$

13. An antibody according to any one of claims 4 to 12 which is a monoclonal antibody.

14. A process for the preparation of a polypeptide according to claim 1 characterized in that conventional polypeptide synthesis methods are used.

15. A process according to claim 14 characterized in that solid phase synthesis methods are used.

16. A process for the preparation of an immunogenic composition according to claim 3 characterized in that a polypeptide of claim 1 is directly covalently coupled to an immunogenic carrier material.

17. A process for the preparation of an immunogenic composition according to claim 3 characterized in that a polypeptide of claim 1 is coupled to an immunogenic carrier material via a conventional bifunctional linking group.

18. A process for the preparation of an antibody as claimed in any one of claims 4 to 12 characterized in that an immunogenic composition according to claim 3 is injected into a host, capable of eliciting an immune response against the ras polypeptide of the immunogenic composition.

19. An immunometric assay for a combination of two or more human p21ras proteins selected from the group consisting of p21ras$^H$, p21ras$^N$, p21ras$^{K4A}$ and p21ras$^{K4B}$ in a test sample characterized in employing antibodies selective for the human p21ras proteins to be assayed, said antibodies being non-crossreactive with the other human p21ras proteins.

20. An immunometric assay for a human p21ras protein selected from the group consisting of p21ras$^H$, p21ras$^N$, p21ras$^{K4A}$ and p21ras$^{K4B}$ in a test sample characterized in employing an antibody selective for the human p21ras protein to be assayed, said antibody being non-crossreactive with the other human p21ras proteins.

21. An immunometric assay according to claim 20 which is a heterogeneous assay.

22. An immunometric assay according to claim 21 wherein an immunoblot procedure is employed, wherein the presence of an immune complex formed between a human p21ras protein to be assayed in the test sample and a selective antibody is determined by contacting said immune complex with a labelled second antibody.

23. An immunometric assay according to claim 22 wherein the second antibody is labelled with an enzyme and a substrate for such enzyme is added, said substrate being converted by said enzyme from a non-colored to a colored state.

24. An immunometric assay according to claim 20 which is a homogeneous assay.

25. An immunometric assay according to claim 24 which is a competitive inhibition assay utilizing a known amount of a labelled human p21ras protein or carboxy terminal fragment thereof corresponding to the human p21ras protein to be assayed.

26. A process for the purification of a specific human p21ras protein selected from the group consisting of p21ras$^H$, p21ras$^N$, p21ras$^{K4A}$ and p21ras$^{K4B}$ characterized in that the purification is done using an antibody selective for the human p21ras protein to be purified and non-crossreactive to other members of the human p21ras protein family bound to an affinity chromatography matrix.

27 Use of an immunogenic composition according to claim 3 for eliciting an immune response in a host.

28. Use of an antibody according to any one of claims 4 to 13 for an immunometric assay.

29. Use of an antibody according to any one of claims 4 to 13 for the purification of a human p21ras protein or polypeptide comprising an amino acid sequence according to claim 1.

30. Use of an immunometric assay according to any one of claims 19 to 25 for assaying a specific human p21ras protein or polypeptide comprising an amino acid sequence according to claim 1.

31. Immunogenic composition according to claim 3 suitable for eliciting an immune response in a host.

32. Antibody according to any one of claims 4 to 13 suitable for use in an immunometric assay for a human p21ras protein.

***